# EUROPEAN PATENT APPLICATION

(11) **EP 1 994 924 A1**
(43) Date of publication of application: **26.11.2008**
(21) Application number: 07023639.3
(22) Date of filing: 06.12.2007
(51) Int. Cl.: A61K 9/00, A61K 36/16, A61K 36/28, A61K 36/53

(54) **A compound for treating, either in a natural or synthetic manner, bacterial or viral inflammatory pathologies of the throat, in the presence of xerostomy**

(30) Priority: 24.05.2007 IT MI20071051
(71) Applicant: Farmaceutici S.R.L., 15057 Tortona, Alessandria (IT)
(72) Inventor: Bonabello, Elvio, 15057 Tortona (Alessandria) (IT)
(74) Representative: Cicogna, Franco

(57) **Abstract**

A compound for treating, either in a natural or synthetic manner, bacterial or viral inflammatory pathologies of the throat, either in the presence or not of xerostomy (dryness of the jaws) comprises a compound solution characterized in that said solution includes sea water, as a solution solvent, and Betain, as a tissue hydrating substance.

## Description

### BACKGROUND OF THE INVENTION

The present invention relates to a compound for treating, either in a natural or synthetic manner, bacterial or viral inflammatory pathologies of the throat, either in the presence xerostomy (an insufficient salivation) or not.

As is known, laryngitis and pharyngitis are two greatly irritating pathologies, both in adult persons and in children.

The facility therewith a lot of persons, particularly affected by such a disease, contract this ailment during a year, has brought the Applicant to provide a natural formulation, which does not generate addiction problems, even in a case of repeated treatments, but which is also very efficient and novel, with respect to like present commercially available compounds.

A sore throat, as it is improperly defined by affected patients, is a trouble the seriousness of which can vary from a symptomatology of a slight type up to an acute pain, with a related dryness of the jaws and a great swallowing difficulty.

The Italian pharmaceutical market is characterized by a very broad range of products or drugs which have been specifically designed and/or formulated for recovering an affected patient from the above ailments.

The most part of available drugs or compounds comprise, as an active principle, a quaternary ammonium salt consisting of benzalconium or dequalinium chloride which, owing to a local disinfecting action is suitable, within three or four days, to positively solve the problem.

Other products, of a more recent generation, are designed for associating naturally derived substances, or molecules able of providing anaesthetizing capabilities, of a topical type, such as lidocain.

The mentioned compounds, even if they provide a lenitive action on the most evident symptoms, can however provide an early recovering feeling, inducing the patient to suspend, in a great hurry, the treatment, thereby generating a possible bacterial resistance to antibiotics.

Homeopathic products, as well as phytotherapeutic compositions having the same above disclosed functions are moreover known on the market.

However, a problem which has been never addressed in designing or form relating the above mentioned compounds, is related to the fact that, nearly always, the above mentioned cold pathologies are bound or related to a very diffuse tissue involvement, also affecting the high treating or respiratory ways by long duration and resistant rhinites.

In such a case, the respiratory difficult, due to the clogging of the nose respiratory tracts, induces the patients to self-aid by breathing through their mouth.

Thus, in a short time, the production of saliva by the patient salivary glands becomes insufficient to compensate for the increased evaporation of the salivary liquid.

Thus, a very important consequence is that of the so called "dryness" of the jaws, which can favor an increased infection with the consequence that it is necessary to treat the patient with a suitable antibiotic therapy, through a systemic way, or may also cause a further swallowing difficulty, with consequent great feeding difficulties.

### SUMMARY OF THE INVENTION

Thus, in the light of the disclosed problems, the aim of the present invention is to provide such a compound or composition adapted to solve the above mentioned problems.

Within the scope of the above mentioned aim, a main object of the invention is to provide such a compound or composition having a very high antibacteric, anti-viral and anti-microbical activity.

Another object of the present invention is to provide such a compound which is adapted to highly stimulate the patient local immune defenses, thereby providing an improved and quicker recovery.

Yet another object of the present invention is to provide such a compound for hydrating the laryngs-pharyngs , the activity of which is useful not only in that disease which is conventionally called "sore throat", but also in salivation disfunctions related to other pathologies (such as the jaw dryness due to pharmacological treatments, the Sjogren syndrome and so on).

In this connection it should be pointed out that products are already known for treating the aetiologic factors, but they have not been found to provide a lenitive action on the symptomatology.

According to one aspect of the present invention, the above mentioned aim and objects, as well as yet other objects, which will become more apparent hereinafter, are achieved by a compound for treating, either in a natural or synthetic manner, bacterial or viral inflammatory pathologies of the throat, either in the presence or not of xerostomy (the dryness of the jaws), which compound is characterized in that it comprises a solution including a sea water as a solution solvent.

Further characteristics and advantages of the present invention will become more apparent hereinafter from the following detailed disclosure of the compound or composition according to the present invention, which disclosure is herein made only by way of an example of an embodiment of the invention, in order to better clarify the invention itself.

It should be apparent that several compound variations and modifications would be possible, all of which will come within the scope of the invention.

The main characteristic of the present invention is that of using isotonic sterile sea eater, as a solvent of the compound active principles, instead of denaturated or deionized water, or a physiologic solution, as it is conventionally provided in prior like compounds or products.

Sea water, used in the present invention, provides a lot of useful functions.

At first, sea water has a very high disinfecting properties.

Thus, in a fully natural product, specifically designed for frequent applications thereof, a repeated contact of the throat bacteric charge with a sea water solution, even if it has been diluted to present isotonic properties, greatly reduces its proliferating or growth speed, thereby improving the efficiency of the active principles contained in the compound.

Moreover, sea water comprises, dissolved herein, in an ionic form and, accordingly, with a high bioavailability characteristic, nearly all the salts and minerals which are present in the natural field, such as copper, zinc, manganese and selenium.

Actually, the first three elements, actively affect the production of enzymes involved in immune cellular reactions, to provide a very important and useful immune-stimulating action.

Selenium, per se, provides an antiradical function, to hinder a main production of free radicals generated by the inflammatory disease.

Moreover, the presence in sea water of all the above mentioned dissociated ions, provides said sea water with a function of a "natural nourishing integrating element", for efficiently nourishing the cells, which would be very useful for regular physiologic functions.

With respect to the specific hydrating action on the involved throat , negatively affected by the inflammatory condition, according to the invention, beatin has been further added.

This component of the inventive compounds, as it had been demonstrated in laboratory tests, has been found to provide a very high hydrating effect or capability on animal .

In this connection, it should be pointed out that betain or trimethyl glycine is a natural component which has been found to have not only unique properties in hydrating mucouses, but also a very important capability of reduction redness due to components included in several cosmetic compositions, such as, for example, alcohol contained in mouthwashes or sodium lauryl sulphate contained in tooth pastes.

Accordingly, in addition to a marked rehydrating capability of betain, as above mentioned, this substance included in the inventive compound positively contributes also as an active principle for reducing the inflammatory condition.

Among the hydrating substances mostly used for a topical use, is also included the hyaluronic acid; this component, however, has been rejected by formulating operators, since, though it is an optimum substance for embedding and releasing water, it has comparatively high viscosity characteristics, which are much higher than those of betain and would be susceptible to make excessively moistening and slippering the mouth inner walls, with a consequent excessive slipperiness of the tongue.

In this connection it should be pointed out that patients affected by xerostomy have, normally, a high mutagenic streptococcus and lactobacillus rate, a greater trend to carries, a reduction of taste perception and are much more susceptible to oral candidosis.

Mucosal tissues are nourished by sea water salts and ions and hydrated owing to the provision of betain.

Moreover, the compound of the present invention also includes substances, also of a natural original, selected because of their marked antibacteric activity, thereby freatly hindering a proliferation of the infective agents which are present in situ.

To that end, propolis has been selected.

Actually, propolis provides the great advantage that it can be electively used in pathologies of the oral cavity, with satisfactory results.

The bacteriostatic activity has been tested on the inhibition both of the growth of Gram+ strains and Gram- strains, and is due both to the presence of flavonoids and synergy of phenol acid, such as Galingin, Pinocembrin, bezoic acid, pherulic acid, and other bacterial growth inhibiting substances.

The antiviral properties should be searched in propolis hydrosoluble components, which have been found to have an inhibitory function on the growth of Herpes Simplex and Corona Virus and, mainly, on viral infections, such as flu either of type A or type B.

The antimicrobic action should be ascribed to the presence of pinocembrin and pinobankin, in a combined action with caffeic acid and benzyl P-cumarate.

The anaesthetic and healing properties have been experimentally recognized.

At present, intensive studies are moreover performed on propolis antioxidating properties.

For a quicker and more intensive therapeutic activity of the compound according to the present invention, a further natural component has been introduced herein, adapted to provide a deinflammatory function on oral-pharyngeal mucouses.

To that end, Echinacea has been selected.

In fact, from time immemorial, North American Indians already used extracts of ruts of this plant, of which several species are available.

The chemical components providing the pharmacologic activity of Echinacea are: high molecular weight polysaccharides (echinacines), derived from caffeic acid (cinarin, chicoric acid, echinacoside, chlorogenic acid), sesquiterpenic lactones, pyrolizidinic alkaloids, aichilamides, flavonoids and an essential oil.

The pharmacologic properties deriving from the above mentioned substances are mainly of an antiinflammatory and immunostimulating nature.

This component of the compound according to the present invention represents an additional integrating substance for mitigating local inflammations deriving from an infective status, which represent the starting factor of the pathology.

From the above disclosure, it should be apparent that the invention fully achieves the intended aim and objects.

In particular, the fact is to be pointed out that the invention has provided a composition based on a use of two particular substances: one having a nourishing function, that is sea water, and the other having a hydrating function, that is betain.

The compound of invention further includes, as a completing and useful substance, propolis and Echinacea.

The thus formulated compound is susceptible to several modifications and variations, all coming within the scope of the inventive idea.

Moreover, all the component substances can be replaced by other substances having equivalent characteristics.

## Claims

1. A compound for treating, either in a natural or synthetic manner, bacterial or viral inflammatory pathologies of the throat, either in the presence or not of xerostomy (the dryness of jaws), **characterized in that** said compound comprises a compound solution including a sea water as a solution solvent.

2. A compound according to claim 1, **characterized in that** said solvent is an isotonic, and hypotonic or an hypertonic solvent.

3. A compound according to claims 1 and 2, **characterized in that** said solvent has a tissue nourishing activity.

4. A compound according to claim 1, **characterized in that** said compound comprises betain as a tissue hydrating substance.

5. A compound according to claim 4, **characterized in that** said betain is present in said compound in a rate variable from 1 to 20%.

6. A compound according to one or more of the preceding claims, **characterized in that** said compound comprises a dry extract, both of alcoholic and analcoholic nature, of propolis in a rate variable from 2 to 40%.

7. A compound according to one or more of the preceding claims, **characterized in that** said compound further comprises Echinacea, in a form of a dry extract, both of distilled water of a mother dying substance of the Echinacea Angustifolia and/or E. Purpurea and/or E. Pallida type.

8. A compound according to one or more of the preceding claims, **characterized in that** in said compound the rate of Echinacea varies from 0.5 to 20%.

9. A compound according to one or more of the preceding claims, **characterized in that** said compound has several pharmaceutical formulations such as spray lotion, syrup, tablets or pills to be dissolved in the mouth.

10. A compound according to one or more of the preceding claims, **characterized in that** said compound comprises one or more of the disclosed and/or illustrated characteristics.
